(19) Europäisches Patentamt
European Patent Office
Office européen des brevets

(11) EP 2 067 860 A1

(12) EUROPEAN PATENT APPLICATION
published in accordance with Art. 153(4) EPC

(43) Date of publication:
**10.06.2009 Bulletin 2009/24**

(21) Application number: **07828261.3**

(22) Date of filing: **19.09.2007**

(51) Int Cl.:
***C12N 15/09*** (2006.01) ***C12Q 1/68*** (2006.01)
***A01H 1/04*** (2006.01)

(86) International application number:
**PCT/JP2007/068166**

(87) International publication number:
**WO 2008/035702 (27.03.2008 Gazette 2008/13)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **20.09.2006 JP 2006254607**

(71) Applicant: **Sapporo Breweries Limited**
**Tokyo 150-8522 (JP)**

(72) Inventors:
- **OKADA, Yukio**
  **Tokyo 150-8522 (JP)**
- **KOIE, Koichiro**
  **Tokyo 150-8522 (JP)**

(74) Representative: **Vossius & Partner**
**Siebertstrasse 4**
**81675 München (DE)**

# (54) METHOD FOR SELECTION OF HOP STRAIN, BLEEDING MARKER FOR USE IN SELECTION OF HOP STRAIN, AND PRIMER SET

(57) It is an object of the invention to screen for hop varieties with high $\alpha$ acid contents, as well as hop varieties with high contents of $\alpha$ acids, $\beta$ acids, myrcene and/or xanthohumol in addition to $\alpha$ acids, within a short time period utilizing a molecular screening method that employs a breeding marker. The invention provides a breeding marker represented by the following (a) or (b), which is used for screening of hop varieties with high $\alpha$ acid contents.

(a) A polynucleotide consisting of a nucleotide sequence of 20-1587 continuous nucleotides including nucleotide No.899 of the polynucleotide consisting of the nucleotide sequence as set forth in SEQ ID NO: 5 (wherein the 899th nucleotide is t).
(b) A polynucleotide consisting of the sequence complementary to the polynucleotide of (a) above.

EP 2 067 860 A1

## Description

### Technical Field

The present invention relates to a method for screening of hop varieties and to a breeding marker and primer set to be used for screening of hop varieties.

### Background Art

Hops, the primary material for beer, impart a refreshing bitterness and aroma to beer. The known bittering agents in hops include α acids such as humulone, cohumulone and adhumulone, and β acids such as lupulone, colupulone and adlupulone. The α acids exhibit antibacterial activity, anticancer activity, anti-inflammatory effects and so on, and the β acids exhibit sedative effects and so on, and thus, they are both being considered also as drug sources (Non-patent document 1).

Terpenes are among the aromatic components of hops in beer. Few details are known about the hop terpenes in relation to beer aroma, but myrcene is the main component among the terpenes in hop cones (Non-patent document 2).

Xanthohumol has been discovered as a flavonoid peculiar to hops and has been shown to exhibit various pharmacological effects including cancer cell growth inhibition, antioxidant effects, hypolipidemic effects and osteoclasis inhibition, for which reason it is being considered as a drug source (Non-patent document 1).

The content of α acids, β acids, myrcene and xanthohumol in hops is an important index for judging hop quality, and conventional breeding of hops has placed great importance on screening for hop varieties that contain high levels especially of α acids and xanthohumol.

Methods used for breeding of useful plants include methods in which high-grade varieties are crossed and the obtained hybrid progeny are cultivated until expression of the desired traits and screened, and molecular screening methods in which the genomic DNA of hybrid progeny are examined at the seed or seedling stage and screened based on breeding markers (DNA markers).

[Non-patent document 1] Gerhaeuser, European Journal of Cancer, 2005, Vol. 41, Not.13, p.1941-1954

[Non-patent document 2] Aoshima et al., J. Agric. Food Chem., 2006, Vol. 5, No.54, p.2514-2519

### Disclosure of the Invention

### Problems to be Solved by the Invention

However, virtually no genetic research has been conducted on male hop plants, which produce no cones, and virtually none of the genes involved in biosynthesis of the aforementioned components associated with hop bitterness and aroma have been identified even in female plants. Consequently, it has not been possible to utilize molecular screening methods based on breeding markers, for screening of hop varieties based on content of α acids, β acids, myrcene and/or xanthohumol.

In addition, since α acids, β acids, myrcene and xanthohumol accumulate specifically in the lupulin gland of hop cones, screening of hop varieties with high levels of these components can only be done after seeding and waiting for forming of the cones, and at least several years of cultivation have been necessary for stabilization of these traits.

It is therefore an object of the present invention to utilize a breeding marker-based molecular screening method for screening of hop varieties with high α acid contents, within a shorter time period. It is another object of the invention to screen for hop varieties with high contents of β acids, myrcene and/or xanthohumol in addition to α acids, within a shorter time period.

### Means for Solving the Problems

In order to achieve the objects stated above, the invention provides breeding markers, represented by the following (a) or (b), to be used for screening of hop varieties with high α acid contents.

(a) A polynucleotide consisting of a nucleotide sequence of 20-1587 continuous nucleotides including nucleotide No.899 of the polynucleotide consisting of the nucleotide sequence as set forth in SEQ ID NO: 5 (wherein the 899th nucleotide is t).

(b) A polynucleotide consisting of the sequence complementary to the polynucleotide of (a) above.

The present inventors have discovered the 899th nucleotide (t) of the polynucleotide consisting of the nucleotide sequence as set forth in SEQ ID NO: 5, as a polymorphism found to be common to hop varieties with high α acid contents, from a cDNA library prepared from the lupulin gland of hop cones. No breeding marker for α acids that can be applied to all hops has been reported to date, but it was found that this breeding marker allows screening of hop varieties with high α acid contents among hybrid progeny obtained by crossing various hop lines. Also, it was demonstrated that the hop varieties screened with the breeding marker also have high contents of β acids, myrcene and/or xanthohumol in addition to α acids.

If, during breeding of hop varieties, those hop varieties having the same sequence as the breeding marker are selected, it is possible to screen for hop varieties with high α acid contents at the seed or seedling stage without waiting for the hops to grow and form cones. Moreover, determining the presence of the breeding marker allows objective, genomic information-based identification of the combinations of crossed lines, that has hitherto depended on intuition and experience. The breeding marker may also be suitably used for screening of hop varieties with high contents of β acids, myrcene and/or xanthohumol in addition to α acids.

Here, the phrase "hop varieties with high α acid contents" refers to hop varieties in groups with high average values for α acid content, when groups of hybrid progeny are categorized according to whether or not they have the same sequence as the aforementioned breeding marker. Similarly, the phrase "hop varieties also with high contents of β acid, myrcene and/or xanthohumol" refers to hop varieties in groups with high average values for α acid content as well as β acid, myrcene and/or xanthohumol contents, when groups of hybrid progeny are categorized according to whether or not they have the same sequence as the aforementioned breeding marker.

The invention still further provides a screening method for hop varieties with high α acid contents, comprising an extraction step in which genomic DNA is extracted from a hop variety specimen, a digestion step in which the genomic DNA is digested with restriction enzyme EcoT22I to obtain digested genomic DNA fragments, a detection step in which the digested genomic DNA fragments are separated and genomic DNA fragments that hybridize to the polynucleotide of (a) or (b) above are detected, and a judging step in which it is judged that the variety has a high α acid content if the size of at least one of the genomic DNA fragments is approximately 11.3 kbp.

With this screening method it is possible to screen for hop varieties with high α acid contents or hop varieties with high α acid and high β acid, myrcene and/or xanthohumol contents, by using the polynucleotide of (a) or (b) as a probe and examining Restriction Fragment Length Polymorphisms (RFLP) with restriction enzyme EcoT22I. Since genome fragment sizes can be visually discriminated, it is possible to objectively and rapidly screen for hop varieties with high α acid contents or hop varieties with high α acid and β acid, myrcene and/or xanthohumol contents without examining the nucleotide sequences of the genomic DNA.

The invention yet further provides a method for screening of hop varieties with high α acid contents which comprises an extraction step in which genomic DNA is extracted from a hop variety specimen, and an identifying step in which genomic DNA polymorphism at the 899th nucleotide position of the nucleotide sequence as set forth in SEQ ID NO: 5 is identified.

The identifying step preferably comprises a PCR step in which DNA is synthesized by PCR using the aforementioned genomic DNA as template, a primer consisting of a nucleotide sequence of 18-50 contiguous nucleotides located toward the 5'-end from the 899th nucleotide of the nucleotide sequence as set forth in SEQ ID NO: 5 and a primer consisting of the sequence complementary to a nucleotide sequence of 18-50 contiguous nucleotides located toward the 3'-end from the 899th nucleotide of the nucleotide sequence as set forth in SEQ ID NO: 5, a digestion step in which the DNA is digested with restriction enzyme PshBI to obtain digested DNA fragments, and a judging step in which the variety is judged to have a high α acid content if cleavage occurs between the 899th nucleotide and the 900th nucleotide of the nucleotide sequence as set forth in SEQ ID NO: 5.

In this screening method, the extracted genomic DNA is used as template to amplify the DNA fragment containing the 899th nucleotide of the nucleotide sequence as set forth in SEQ ID NO: 5, and therefore no Southern hybridization is necessary. If the digested DNA fragments obtained in the digestion step are fractionated based on the DNA fragment sizes by electrophoresis, for example, it is possible to easily judge whether they have been cleaved between the 899th nucleotide and the 900th nucleotide of the nucleotide sequence as set forth in SEQ ID NO: 5, thus allowing screening of hop varieties with high α acid contents or hop varieties with high α acid and β acid, myrcene and/or xanthohumol contents.

The identifying step preferably comprises a PCR step in which DNA is synthesized by PCR using the aforementioned genomic DNA as template, a primer including the nucleotide sequence as set forth in SEQ ID NO: 2 and a primer including the nucleotide sequence as set forth in SEQ ID NO: 3, a digestion step in which the DNA is digested with restriction enzyme PshBI to obtain digested DNA fragments, and a judging step in which the variety is judged to have a high α acid content if the size of at least one of the digested DNA fragments is approximately 460 bp.

In this screening method, since PCR is conducted using the extracted genomic DNA as template, a primer containing the nucleotide sequence as set forth in SEQ ID NO: 2 and a primer containing the nucleotide sequence as set forth in SEQ ID NO: 3, only the target DNA fragment is efficiently and specifically amplified. In addition, if the digested DNA fragments obtained in the digestion step are fractionated based on the DNA fragment sizes, it is possible to easily judge whether an approximately 460 bp fragment is present, thus allowing screening of hop varieties with high α acid contents or hop varieties with high α acid and β acid, myrcene and/or xanthohumol contents.

The invention yet further provides a primer set to be used for screening of hop varieties with high α acid contents, the primer set comprising a primer containing the nucleotide sequence as set forth in SEQ ID NO: 2 and a primer containing the nucleotide sequence as set forth in SEQ ID NO: 3.

Using this primer set allows specific amplification of only the DNA fragment containing the 899th nucleotide of the nucleotide sequence as set forth in SEQ ID NO: 5, using the extracted genomic DNA as template. The primer set can be appropriately utilized for screening of hop varieties with high α acid contents and with high β acid, myrcene and/or xanthohumol contents.

## Effect of the Invention

According to the invention it is possible to provide α breeding marker for hop varieties with high α acid contents, and the breeding marker also can be utilized as a breeding marker for hop varieties with high α acid contents and high β acid, myrcene and/or xanthohumol contents. According to the invention it is also possible to objectively and rapidly screen for hop varieties with high α acid contents or hop varieties with high α acid, myrcene and/or xanthohumol contents, using hop seeds or seedlings, without waiting for the hops to form cones and develop stabilized traits.

## Brief Description of the Drawings

Fig. 1 is an image showing EcoT22I/7H01 polymorphism, obtained by digestion of genomic DNA of hop varieties with restriction enzyme EcoT22I, and genomic Southern hybridization.
Fig. 2 shows homology of the nucleotide sequences of 1-type g7H01 and m-type g7H01.
Fig. 3 shows a typical band pattern for PshBI/g7H01 polymorphism, obtained by PCR-RFLP of hop varieties.
Fig. 4 is an image showing PshBI/g7H01 polymorphism, obtained by analysis of genomic DNA of 11 hop lines.
Fig. 5 is an image showing EcoT22I/7H01 polymorphism, obtained by genomic analysis of 11 hop lines.

## Best Modes for Carrying Out the Invention

Preferred embodiments of the invention will now be described in detail.

The breeding markers of the invention are characterized by being represented by the following (a) or (b), and being used for screening of hop varieties with high α acid contents.

(a) A polynucleotide consisting of a nucleotide sequence of 20-1587 continuous nucleotides including nucleotide No.899 of the polynucleotide consisting of the nucleotide sequence as set forth in SEQ ID NO: 5 (wherein the 899th nucleotide is t).
(b) A polynucleotide consisting of the sequence complementary to the polynucleotide of (a) above.

The term "breeding marker" means a DNA marker used for breeding, and it is a DNA sequence that exists in a genome near a gene associated with a trait of interest and operates together with the gene. A breeding marker allows identification of traits that cannot be distinguished by the naked eye, such as disease resistance, or traits that appear in the mature phase, by using genomic DNA from seeds or seedlings of the plant specimen. The breeding marker may be the gene itself, or DNA other than the gene.

The term "α acid" refers to a bitter substance in hops that is primarily humulone and its analogs including cohumulone, adhumulone and so on. The term "β acid" refers to a bitter substance in hops that is primarily lupulone and its analogs including colupulone, adlupulone and so on.

Throughout the present specification, "hops" refers to the perennial plant (Humulus lupulus L.) itself, and "cones" refers to the female flower produced by female hops, where the perichaetium has formed a pine cone-shaped chamber. The term "hop variety" means each hybrid progeny obtained by breeding hops.

The term "polynucleotide" refers to a molecule with a plurality of bonded phosphoric acid esters of nucleosides comprising purine or pyrimidine bases that are β-N-glycoside bonded to sugars (ribonucleotides or deoxyribonucleotides).

The polynucleotides represented by (a) or (b) above may be cloned from a hop genomic library using a labeled DNA probe synthesized based on the nucleotide sequence information of SEQ ID NO: 5, according to the method described in, for example, Molecular cloning (Maniatis et al., 1989, Cold Spring Harbor Laboratory Press).

The polynucleotides represented by (a) or (b) may also be synthesized by PCR using a primer set designed based on the nucleotide sequence information of SEQ ID NO: 5, with genomic DNA extracted from tissue of hop leaves or the like as template. Also, partial sequences of the polynucleotide represented by (a) or (b) may be synthesized with a DNA synthesizer and linked by an enzymatic method and subcloning, to obtain a polynucleotide of the target size.

The breeding marker may be used for screening of hop varieties with high α acid contents, but it is preferably used for screening of hop varieties with high α acid contents and high β acid, myrcene and/or xanthohumol contents.

Also, the screening method for hop varieties with high α acid contents according to the invention is characterized by comprising an extraction step in which genomic DNA is extracted from a hop variety specimen, a digestion step in which the genomic DNA is digested with restriction enzyme EcoT22I to obtain digested genomic DNA fragments, a detection step in which the digested genomic DNA fragments are separated and genomic DNA fragments that hybridize to the polynucleotide of (a) or (b) above are detected, and a judging step in which it is judged that the variety has a high α acid content if the size of at least one of the genomic DNA fragments is approximately 11.3 kbp.

Methods utilizing RFLP may be mentioned as examples of such screening methods. Specifically, this method compares the sizes of DNA fragments produced after treatment with a restriction enzyme, based on mutations or nucleotide insertions or deletions at a restriction enzyme recognition site in the genomic DNA, to determine whether or not a certain trait is present.

In the detection step, it is possible to detect genomic DNA fragments that hybridize to the polynucleotides represented by (a) or (b) by genomic Southern hybridization, as described in Molecular cloning (Maniatis et al., 1989, Cold Spring Harbor Laboratory Press), for example. Specifically, the digested genomic DNA fragments of a hop specimen digested with restriction enzyme EcoT22I are fractionated by agarose gel electrophoresis, the fractionated genomic DNA fragments are transferred to a nylon membrane, a labeled polynucleotide represented by (a) or (b) above is used as a probe for hybridization, and the hybridized bands are detected based on the probe labeling.

The probe labeling method may be a method in which a DNA polymerase such as Klenow enzyme is used to incorporate substrate nucleotides that are labeled with an isotope such as $^{32}P$, a fluorescent dye, digoxigenin (DIG) or biotin, using random hexamer oligonucleotides and the like as primer (random primer method), or a method in which T4 polynucleotide kinase is used for phosphorylation of the 5'-end of the oligonucleotide with $^{32}P$ or the like for labeling.

In the judging step, digested genomic DNA fragments from a hop variety specimen may be electrophoresed with a DNA size marker and transferred to a nylon membrane, with the well positions and size marker migration positions marked on the nylon membrane, thus allowing the sizes of the digested genomic DNA fragments from hybridized hop variety specimens to be calculated based on the relationship between migration distance from the well and DNA size marker size. If a digested genomic DNA fragment of approximately 11.3 kbp is detected in the hop variety specimen, the hop variety specimen may be judged to be a variety with a high α acid content.

The method for screening of hop varieties with high α acid contents according to the invention is also characterized by comprising an extraction step in which genomic DNA is extracted from a hop variety specimen, and an identifying step in which genomic DNA polymorphism at the 899th nucleotide position of the nucleotide sequence as set forth in SEQ ID NO: 5 is identified.

The identifying step preferably comprises a PCR step in which DNA is synthesized by PCR using the aforementioned genomic DNA as template, a primer consisting of a nucleotide sequence of 18-50 contiguous nucleotides located toward the 5'-end from the 899th nucleotide of the nucleotide sequence as set forth in SEQ ID NO: 5 and a primer consisting of the sequence complementary to a nucleotide sequence of 18-50 contiguous nucleotides located toward the 3'-end from the 899th nucleotide of the nucleotide sequence as set forth in SEQ ID NO: 5, a digestion step in which the DNA is digested with restriction enzyme PshBI to obtain digested DNA fragments, and a judging step in which the variety is judged to have a high α acid content if cleavage occurs between the 899th nucleotide and the 900th nucleotide of the nucleotide sequence as set forth in SEQ ID NO: 5.

Methods utilizing PCR-RFLP may be mentioned as examples of such a screening method. Specifically, such methods compare the sizes of DNA fragments produced after treatment with a restriction enzyme, based on mutations or nucleotide insertions or deletions at a restriction enzyme recognition site in the genomic DNA or a DNA fragment amplified by PCR using cDNA as template, to determine whether or not a certain trait is present.

The primer used in the PCR step may be synthesized with a DNA synthesizer, selecting a nucleotide sequence of 18-50 contiguous nucleotides from the nucleotide sequence as set forth in SEQ ID NO: 5. The size of the primer is preferably at least 18 and no greater than 30 nucleotides, and more preferably at least 20 and no greater than 22 nucleotides.

In the judging step, digested DNA fragments from a hop variety specimen digested with restriction enzyme PshBI may be fractionated by agarose electrophoresis or polyacrylamide electrophoresis together with a DNA size marker, thus allowing calculation of the sizes of the digested DNA fragments from hop variety specimens based on the relationship between migration distance of the DNA size marker from the well and size marker nucleotide sequence size. If the sizes of the digested DNA fragments from the hop variety specimen are known, then it is possible to easily judge whether they have been cleaved between the 899th nucleotide and the 900th nucleotide of the nucleotide sequence as set forth in SEQ ID NO: 5, so that the hop variety specimen can be judged as a variety with a high α acid content when cleavage has been confirmed. The digested DNA fragments and size marker in the gel after electrophoresis may be detected under LAV irradiation after treatment with, for example, ethidium bromide or SYBR Green.

Alternatively, the identifying step preferably comprises a PCR step in which DNA is synthesized by PCR using genomic DNA from a hop variety specimen as template, a primer including the nucleotide sequence as set forth in SEQ ID NO: 2 and a primer including the nucleotide sequence as set forth in SEQ ID NO: 3, a digestion step in which the DNA is digested with restriction enzyme PshBI to obtain digested DNA fragments, and a judging step in which the variety is judged to have a high α acid content if the size of at least one of the digested DNA fragments is approximately 460 bp.

The primer used in the PCR step may be synthesized with a DNA synthesizer based on the nucleotide sequence as set forth in SEQ ID NO: 2 and the nucleotide sequence as set forth in SEQ ID NO: 3.

In the judging step, digested DNA fragments from a hop variety specimen digested with restriction enzyme PshBI may be fractionated by agarose electrophoresis or polyacrylamide electrophoresis together with a DNA size marker, thus allowing calculation of the sizes of the digested DNA fragments from hop variety specimens based on the relationship between migration distance of the DNA size marker from the well and size marker nucleotide sequence size. As a result, it is possible to determine that a hop variety specimen is a variety with a high α acid content if a digested DNA fragment of approximately 460 bp is detected in the hop variety specimen. The digested DNA fragments and size marker in the gel after electrophoresis may be detected under UV irradiation after treatment with, for example, ethidium bromide or SYBR Green.

The screening method of the invention may be used for screening of hop varieties with high α acid contents, but it is preferably used for screening of hop varieties with high α acid contents and high β acid, myrcene and/or xanthohumol contents.

The primer set of the invention is a primer set to be used for screening of hop varieties with high α acid contents, characterized by comprising a primer containing the nucleotide sequence as set forth in SEQ ID NO: 2 and a primer containing the nucleotide sequence as set forth in SEQ ID NO: 3.

The primer set may be synthesized with a DNA synthesizer based on the nucleotide sequence as set forth in SEQ ID NO: 2 and the nucleotide sequence as set forth in SEQ ID NO: 3.

The primer set may be used for screening of hop varieties with high α acid contents, but it is preferably used for screening of hop varieties with high α acid contents and high myrcene and/or xanthohumol contents.

## Examples

The present invention will now be explained in greater detail with reference to examples, with the understanding that the invention is not meant to be limited to these examples.

### 1. Experiment method

#### 1) Quantitation of α acids, β acids and xanthohumol in hop cones:

The quantities of α acids, β acids and xanthohumol in the hop cones were measured by a modified mode of the official method of the American Society of Brewing Chemists (ASBC). Specifically, 1 g of pulverized hop cones was first placed in a 100 mL stoppered Erlenmeyer flask, and then 4 mL of methanol and 20 mL of diethyl ether were added prior to shaking for 30 minutes. Next, 8 mL of 0.1 M hydrochloric acid was added prior to shaking for 10 minutes, and after standing, 0.2 mL of the ether layer was sampled, methanol was added to a volume of 10 mL and a portion was analyzed by high performance liquid chromatography (HPLC). The HPLC conditions were based on Official Method 7.7 of the European Brewery Convention (EBC), and the α acids, β acids and xanthohumol were detected and quantified at a wavelength of 314 nm.

#### 2) Quantitation of myrcene in hop cones:

The quantity of myrcene in hop cones was determined by the following procedure. First, 1 g of pulverized hop cones was placed in a 10 mL stoppered centrifuge tube, and then 6 mL of n-hexane was added prior to shaking for 45 minutes, after which the mixture was centrifuged at 3,000 rpm, 20°C for 10 minutes and 1.5 mL of the supernatant was sampled in a 10 mL stoppered centrifuge tube. Next, 3 mL of 5% potassium carbonate was added prior to shaking for 5 minutes, after which the mixture was centrifuged at 3,000 rpm, 20°C for 10 minutes and the supernatant was analyzed by gas chromatography. The gas chromatography was carried out with an HP5890 by Hewlett Packard, the column was a DB-1 (0.25 mm × 30 m × 1.0 μm) by J&W and the detection system was flame ionization detection (FID). The carrier gas was helium (2.2 mL/min), and the temperature program for the oven was set to 1 minute warming at 50°C followed by heating from 50°C to 250°C at 5°C/min and warming at 250°C for 3 minutes.

3) Preparation of total RNA and poly $A^+$ RNA from lupulin gland of hop cones:

First, the frozen cones of female hop plants were crushed and the fraction that passed through a 250 μm mesh diameter sieve was used as the lupulin gland fraction. The lupulin gland fraction was then homogenized in liquid nitrogen, a 2% CTAB solution (2% cetyltrimethylammonium bromide, 20 mM EDTA, 1.4 M NaCl, 5% β-mercaptoethanol, 0.1 M Tris, pH 9.5) was added to create a suspension, and after warming at 65°C for 10 minutes, extraction was performed twice with chloroform/isoamyl alcohol (24:1). A 1/3-fold amount of 10 M lithium chloride was added to the obtained water-soluble fraction, and the mixture was allowed to stand overnight and then centrifuged at 15,000 rpm for 10 minutes, after which the obtained precipitate was dissolved in RNase-free sterilized water. Next, a 1/3-fold amount of 10 M lithium chloride was further added to the resulting solution, and the mixture was allowed to stand overnight and then centrifuged at 15,000 rpm for 10 minutes, after which the obtained precipitate was dissolved in RNase-free sterilized water for use as the total RNA sample.

The obtained total RNA sample was quantitated by measurement of the absorbance at 260 nm using a spectrophotometer, and then Oligotex-dT30 Super by Takara Bio, Inc. was used for preparation of poly $A^+$ RNA following the manufacturer's protocol.

4) Preparation of hop cone lupulin gland cDNA library:

A Creator SMART cDNA Library Construction Kit by BD Biosciences was used to prepare a hop cone lupulin gland cDNA library from the poly $A^+$ RNA obtained from hop cone lupulin gland. The procedure for preparation of the cDNA library was according to the manufacturer's protocol supplied with the kit.

2. Experimental results

(Example 1) Search for DNA markers related to α acid, β acid and myrcene contents

First, clones were randomly selected from a hop cone (Saaz variety) lupulin gland cDNA library, and lupulin gland cDNA introduced into each clone was used as template for PCR in the presence of digoxigenin (DIG)-labeled dUTP, to create DIG-labeled probes. The DIG-labeled probes were created by PCR using a PCR DIG probe synthesis kit by Roche Diagnostics K.K.

Next, genomic DNA was extracted from 110 hybrid progeny of Chinook and SaM hop varieties stocked by Sapporo Breweries Ltd. and analyzed by RFLP using combinations of the aforementioned DIG-labeled probes and restriction enzymes EcoT22I and XbaI, and a search was conducted for breeding markers that can detect polymorphisms correlated with α acid, β acid, myrcene and xanthohumol contents. The genomic DNA extraction and genomic Southern hybridization were conducted according to the method described in Molecular cloning (Maniatis et al., 1989, Cold Spring Harbor Laboratory Press).

As a result, polymorphism detected with the combination of the cDNA clone 7H01 (hereinafter "7H01") from lupulin gland and restriction enzyme EcoT22I (hereinafter, "EcoT22I/7H01 polymorphism") was shown to be correlated with α acid, β acid, myrcene and xanthohumol contents, suggesting that it can be utilized as a breeding marker.

In Example 2 it was examined whether 7H01 call be utilized as a breeding marker correlated with α acid, β acid, myrcene or xanthohumol contents in a wide range of hop lines other than the hybrid progeny of the Chinook and SaM varieties. The nucleotide sequence of 7H01 is listed as SEQ ID NO: 1.

(Example 2) Correlation between α acid, β acid, myrcene or xanthohumol content and EcoT22I/7H01 polymorphism:

Eleven hop lines stocked by Sapporo Breweries Ltd. (Daimanshu, M916001003, 0004B, 971174, 980573, Monica, M843501246, 980690, Hokusenzairai, Maria, 981116) were selected and crossbred to obtain hybrid progeny, the α acid, β acid, myrcene and xanthohumol contents of the hop cones were examined, and their correlation with EcoT22I/7H01 polymorphism was determined.

The cross breeding was carried out with the combinations Daimanshu × M916001003, 0004B × 971174, 0004B × 980573, Monica x M843501246, Monica × 980573, Daimanshu × 980690, Hokusenzairai × 980690 and Maria × 981116, and the α acid, β acid, myrcene and xanthohumol contents in the cones of the obtained hybrid progeny were examined.

Correlation between the EcoT22I/7H01 polymorphism and α acid, β acid, myrcene or xanthohumol content was judged by dividing the hybrid progeny obtained by cross breeding into groups based on EcoT22I/7H01 polymorphism, calculating the mean values for the component contents in each group, and determining statistically significant difference by T test. For the T test, the difference in variance of measured values among the EcoT22I/7H01 polymorphisms was investigated by F test for each hop line combination, and homoskedasticity was assumed when no difference in variance was found, while variance was assumed to be unequal when a difference in variance was found. The contribution ratio representing the effect of 7H01 on α acid, β acid, myrcene or xanthohumol content was calculated as genetic variance/ population variance. The genetic variance was the dispersion for a theoretical population experiencing no factors other than the subject genotype 7H01 (no influence by other gene loci or environment). Specifically, if the mean value for the parent population is represented as XP, the population variance as VP, the mean value for a population with genotypes 1, 2, ... as X1, X2, ... and the number of individuals in each genotype as N1, N2, then the genetic variance ($V_G$) and contribution ratio (H) may be calculated by the following formulas.

$$\text{Genetic variance } (V_G) = \left( (X_P - X_1)^2 \times N_1 + (X_P - X_2)^2 \times N_2 + \ldots \right) / (N_1 + N_2 + \ldots)$$

$$\text{Contribution ratio } (H) = V_G / V_P$$

Fig. 1 is an image showing EcoT22I/7H01 polymorphism, obtained by digestion of genomic DNA of different hop varieties with restriction enzyme EcoT22I, and genomic Southern hybridization. The hybrid progeny obtained by the different cross breeding combinations exhibited three different patterns, ll-type, lm-type and mm-type.

Table 1 and Table 2 show the correlation between EcoT22I/7H01 polymorphism and α acid content. Table 1 shows the combinations with the polymorphism patterns of the hybrid progeny separated into ll-type and lm-type, and Table 2 shows the combinations separated into lm-type and mm-type. The α acid contents are shown as ratios of α acid weight in the cones with respect to dry weight of the cones.

[Table 1]

| Breeding combination | Number of hybrid progeny | | α-Acid content (%) | | | Contribution ratio (%) |
|---|---|---|---|---|---|---|
| | ll | lm | ll | lm | Significant difference | |
| Daimanshu (lm) × M916001003 (ll) | 17 | 8 | 4.89 | 7.00 | * | 20.86 |
| 0004B (lm) × 971174 (ll) | 16 | 23 | 8.76 | 9.11 | N.S. | 0.31 |
| 0004B (lm) × 980573 (ll) | 21 | 14 | 8.19 | 11.51 | ** | 38.62 |
| Monica (lm) × M843501246 (ll) | 20 | 19 | 7.33 | 9.32 | ** | 24.29 |
| Monica (lm) × 980573 (ll) | 27 | 23 | 9.15 | 10.91 | ** | 17.25 |

* : Significance level ≤5%, Significant difference
** : Significance level ≤1%, Significant difference
N.S.: Not significant

[Table 2]

| Breeding combination | Number of hybrid progeny | | α-Acid content (%) | | | Contribution ratio (%) |
|---|---|---|---|---|---|---|
| | lm | mm | lm | mm | Significant difference | |
| Daimanshu (lm) × 980690 (mm) | 18 | 12 | 4.84 | 4.41 | N.S. | 1.18 |
| Hokusenzairai (lm) × 980690 (mm) | 19 | 14 | 4.14 | 4.91 | N.S. | 3.08 |
| Maria (lm) × 981116 (mm) | 31 | 8 | 10.27 | 10.29 | N.S. | 0.001 |

As a result of comparison of the α acid contents in each group of hybrid progeny of combinations divided into ll-type and lm-type (Table 1), the α acid contents were higher in the groups with the lm-type for all of the breeding combinations, with statistical significance ($p < 0.05$) in 4 of the 5 breeding combinations. On the other hand, when the α acid contents are compared in each group of hybrid progeny of combinations divided into lm-type and mm-type (Table 2), virtually no difference was found between groups, nor was any statistically significant difference found.

These results suggested that a hop variety containing at least one m-type genome has a higher α acid content than a hop variety without an m-type genome.

Table 3 and Table 4 show the correlation between EcoT22I/7H01 polymorphism and β acid content. Table 3 shows the combinations with the polymorphism patterns of the hybrid progeny separated into ll-type and lm-type, and Table 4 shows the combinations separated into lm-type and mm-type. The β acid contents are shown as ratios of β acid weight in the cones with respect to dry weight of the cones.

[Table 3]

| Breeding combination | Number of Hybrid progeny | | β-Acid content (%) | | | Contribution ratio (%) |
|---|---|---|---|---|---|---|
| | ll | lm | ll | lm | Significant difference | |
| Daimanshu (lm) × M916001003 (ll) | 17 | 8 | 2.44 | 3.64 | ** | 22.24 |
| 00048 (lm) × 971174 (ll) | 16 | 23 | 2.84 | 2.88 | N.S. | 0.05 |
| 0004B (lm) × 980573 (ll) | 21 | 14 | 3.99 | 4.53 | N.S. | 5.84 |
| Monica (lm) × M843501246 (ll) | 20 | 19 | 3.27 | 4.39 | ** | 18.89 |
| Monica (lm) × 980573 (ll) | 27 | 23 | 4.59 | 5.24 | N.S. | 4.73 |

** : Significance level ≤1%, Significant difference
N.S.: Not significant

[Table 4]

| Breeding combination | Number of Hybrid progeny | | β-Acid content (%) | | | Contribution ratio (%) |
|---|---|---|---|---|---|---|
| | lm | mm | lm | mm | Significant difference | |
| Daimanshu (lm) × 980690 (mm) | 18 | 12 | 4.00 | 3.48 | N.S. | 3.82 |
| Hokusenzairai (lm) × 980690 (mm) | 19 | 14 | 2.95 | 4.00 | ** | 26.93 |
| Maria (lm) × 981116 (mm) | 31 | 8 | 3.16 | 3.49 | N.S. | 4.71 |

**: Significance level ≤1%, Significant difference
N.S.: Not significant

As a result of comparison of the β acid contents in each group of hybrid progeny of combinations divided into ll-type and lm-type, the β acid contents were higher in the groups with the lm-type for all of the breeding combinations, with statistical significance ($p < 0.05$) in 2 of the 5 breeding combinations. On the other hand, comparison of the β acid contents in each group of hybrid progeny of combinations divided into lm-type and mm-type, then acid contents were higher in the groups with the mm-type for 2 of the 3 combinations, with statistical significance ($p < 0.01$) in the Hokusenzairai × 980690 combination. In the remaining combination, however, the β acid content was slightly higher in lm-type groups, and no major difference was found between groups with the lm-type and groups with the mm-type.

These results suggested that a hop variety containing at least one m-type genome has a higher β acid content than a hop variety without an m-type genome.

Table 5 and Table 6 show the correlation between EcoT22I/7H01 polymorphism and myrcene content. Table 5 shows the combinations with the polymorphism patterns of the hybrid progeny separated into ll-type and lm-type, and Table 6 shows the combinations separated into lm-type and mm-type. The myrcene content was represented as the proportion of myrcene weight in the cones with respect to the dry weight of the cones.

[Table 5]

| Breeding combination | Number of Hybrid progeny | | Myrcene content (mg/g cones) | | | Contribution ratio |
|---|---|---|---|---|---|---|
| | ll | lm | ll | lm | Significant difference | (%) |
| Daimanshu (lm) × M916001003 (ll) | 17 | 8 | 2.14 | 2.97 | N.S. | 7.14 |
| 0004 B (lm) × 971174 (ll) | 16 | 23 | 5.36 | 5.86 | N.S. | 0.87 |
| 0004B (lm) × 980573 (ll) | 21 | 14 | 7.08 | 10.97 | ** | 19.67 |
| Monica (lm) × M84350 1246 (ll) | 20 | 19 | 8.00 | 11.71 | ** | 19.93 |
| Monica (lm) × 980573 (ll) | 27 | 23 | 7.84 | 11.29 | ** | 19.82 |

**: Significance level ≤1%, Significant difference
N.S.: Not significant

[Table 6]

| Breeding combination | Number of Hybrid progeny | | Myrcene content (mg/g cones) | | | Contribution ratio |
|---|---|---|---|---|---|---|
| | lm | mm | lm | mm | Significant difference | (%) |
| Daimanshu (lm) × 980690 (mm) | 18 | 12 | 3.03 | 1.88[2] | * | 9.57 |
| Hokusenzairai (lm) × 980690 (mm) | 19 | 14 | 1.73 | 1.97 | * | 0.97 |
| Maria (lm) × 981116 (mm) | 31 | 8 | 9.72 | 6.93 | N.S. | 7.21 |

*: Significance level ≤5%, Significant difference

As a result of comparison of the myrcene contents in each group of hybrid progeny of combinations divided into ll-type and lm-type, the myrcene contents were higher in the groups with the lm-type for all of the breeding combinations, with statistical significance ($p < 0.05$) in 3 of the 5 breeding combinations. On the other hand, comparison of the myrcene contents in each group of hybrid progeny of combinations divided into lm-type and mm-type, the myrcene contents were higher in the groups with the lm-type for 2 of the 3 combinations, with statistical significance ($p < 0.01$) in the Daimanshu × 980690 combination. For the remaining combination, however, the myrcene content was statistically significantly higher in mm-type groups ($p < 0.01$), and no major difference was found between groups with the lm-type and groups with the mm-type.

These results suggested that a hop variety containing at least one m-type genome has a higher myrcene content than a hop variety without an m-type genome.

Table 7 and Table 8 show the correlation between EcoT22I/7H01 polymorphism and xanthohumol content. Table 7 shows the combinations with the polymorphism patterns of the hybrid progeny separated into ll-type and lm-type, and Table 8 shows the combinations separated into lm-type and mm-type. The xanthohumol content was represented as the proportion of xanthohumol weight in the cones with respect to the dry weight of the cones.

[Table 7]

| Breeding combination | Number of Hybrid progeny | | Xanthohumol content (%) | | | Contribution ratio |
|---|---|---|---|---|---|---|
| | ll | lm | ll | lm | Significant difference | (%) |
| Daimanshu (lm) × M916001003 (ll) | 17 | 8 | 0.28 | 0.34 | N.S. | 4.28 |
| 0004B (lm) × 971174 (ll) | 16 | 23 | 0.41 | 0.38 | N.S. | 1.22 |
| 0004B (lm) × 980573 (ll) | 21 | 14 | 0.48 | 0.52 | N.S. | 0.86 |
| Monica (lm) × M843501246 (ll) | 20 | 19 | 0.52 | 0.65 | ** | 17.26 |

(continued)

| Breeding combination | Number of Hybrid progeny | | Xanthohumol content (%) | | | Contribution ratio (%) |
|---|---|---|---|---|---|---|
| | ll | lm | ll | lm | Significant difference | |
| Monica (lm) × 980573 (ll) | 27 | 23 | 0.55 | 0.66 | * | 12.72 |

*: Significance level ≤5%, Significant difference
**: Significance level ≤1%, Significant difference
N.S.: Not significant

[Table 8]

| Breeding combination | Number of Hybrid progeny | | Xanthohumol content (%) | | | Contribution ratio (%) |
|---|---|---|---|---|---|---|
| | lm | mm | lm | mm | Significant difference | |
| Daimanshu (lm) × 980690 (mm) | 18 | 12 | 0.46 | 0.40 | N.S. | 1.95 |
| Hokusenzairai (lm) × 980690 (mm) | 19 | 14 | 0.32 | 0.36 | N.S. | 1.96 |
| Maria (lm) × 981116 (mm) | 31 | 8 | 0.75 | 0.65 | M.S. | 2.99 |

As a result of comparison of the xanthohumol contents in each group of hybrid progeny of combinations divided into ll-type and lm-type, the xanthohumol contents were higher in the groups with the lm-type for 4 of the 5 breeding combinations, with statistical significance ($p < 0.05$) in 2 of the combinations. On the other hand, comparison of the xanthohumol contents in each group of hybrid progeny of combinations divided into lm-type and mm-type, no statistically significant difference was found between the groups with the lm-type and groups with the mm-type for any of the combinations, and no major difference in xanthohumol content was found between the groups.

These results suggested that a hop variety containing at least one m-type genome has a higher xanthohumol content than a hop variety without an m-type genome.

(Example 4) Cloning of genomic DNA fragment containing 7H01

Since it was suggested that a hop variety containing at least one m-type genome has high α acid, β acid, myrcene and xanthohumol contents, the nucleotide sequence of 7H01 was used for cloning of m-type genomic DNA fragments and 1-type genomic DNA fragments, in an attempt to search for polymorphisms correlating with α acid, β acid, myrcene and xanthohumol contents, based on differences in the nucleotide sequences of the two.

First, the following primers were designed based on the sequences at both end regions of 7H01.
p7H01-F primer: 5'-GTTTCCGGAGAATCATGGCG-3' (SEQ ID NO: 2)
p7H01-R primer: 5'-CAAAACTAGTGGAAGAAAGTTCC-3' (SEQ ID NO: 3)

Next, genomic DNA was extracted from Chinook varieties carrying both the 1-type and m-type genomes and a SaM variety carrying only the m-type, PCR was conducted using the p7H01-F primer and p7H01-R primer, and the specifically amplified genomic DNA fragments were cloned. The nucleotide sequences were analyzed for the clones of the DNA fragments cloned from the Chinook variety genomic DNA and the one clone of the DNA fragment cloned from the SaM variety genomic DNA. The PCR was conducted using PrimeSTAR HS DNA Polymerase by Takara Bio, Inc., according to the manufacturer's protocol. The nucleotide sequence analysis was carried out utilizing the Macrogen Data Analysis Service.

Of the nucleotide sequences of the clones of the Chinook variety, those differing from the nucleotide sequence of the clone from the SaM variety were judged to have the nucleotide sequence of the genomic DNA fragment containing the 1-type 7H01 (hereinafter referred to as "1-type g7H01") and those having the nucleotide sequence of the clone from the SaM variety were judged to have the nucleotide sequence of the genomic DNA fragment containing the m-type 7H01 (hereinafter referred to as "m-type g7H01"). The nucleotide sequence of 1-type g7H01 is listed as SEQ ID NO: 4, and the nucleotide sequence of m-type g7H01 is listed as SEQ ID NO: 5.

Fig. 2 shows homology of the nucleotide sequences of 1-type g7H01 and m-type g7H01. According to the results, a single nucleotide polymorphism was found at the 899th nucleotide of m-type g7H01 indicated by the arrow in Fig. 2, and it was demonstrated that this single nucleotide polymorphism can be distinguished by cleavage with restriction enzyme PshBI.

(Example 5) Analysis of single nucleotide polymorphism found between 1-type g7H01 and m-type g7H01

Since it had been determined that m-type g7H01 contains a single nucleotide polymorphism that can be distinguished by cleavage with restriction enzyme PshBI, it was then investigated whether this single nucleotide polymorphism can be utilized to screen for hop varieties with high α, acid, β acid, myrcene and xanthohumol contents, in the same manner as the EcoT22I/7H01 polymorphism.

First, the genomic DNA of 11 hop lines (Daimanshu, M916001003, 0004B, 971174, 980573, Monica, M843501246, 980690, Hokusenzairai, Maria, 981116) were used for PCR-RFLP analysis using a combination of restriction enzyme PshBI and a primer set consisting of p7H01-F primer and p7H01-R primer, and the resulting polymorphism (hereinafter referred to as "PshBI/g7H01 polymorphism") was detected.

Specifically, DNA was synthesized by PCR using the genomic DNA of the 11 hop lines as template with p7H01-F primer and p7H01-R primer, and then the DNA was digested with restriction enzyme PshBI, the digested DNA fragments were fractionated by 1% agarose gel electrophoresis, and the band pattern of the fractionated DNA fragments in the agarose gel was examined. Detection of an approximately 460 bp band and an approximately 680 bp band in analysis of the PshBI/g7H01 polymorphism means that the genomic DNA has the m-type genome, while detection of an approximately 1138 bp band means that the genomic DNA has the 1-type genome.

Fig. 3 shows a typical band pattern for PshBI/g7H01 polymorphism, obtained by PCR-RFLP analysis of hop varieties, and Fig. 4 shows PshBI/g7H01 polymorphism obtained by analysis of the genomic DNA of the 11 hop lines.

The results suggested that 980690 and 981116 are hop lines with only the m-type genome, M916001003, 971174, 980573 and M843501246 are hop lines with only the 1-type genome, and Daimanshu, 0004B, Monica, Hokusenzairai and Maria are hop lines with both the m-type genome and 1-type genome.

Next, genomic DNA from the same 11 hop lines was used for RFLP analysis with a combination of restriction enzyme EcoT22I and 7H01, and the obtained EcoT22I/g7H01 polymorphisms were examined to confirm whether or not the determined results based on PshBI/g7H01 polymorphism were correct.

Specifically, the genomic DNA of each of the 11 hop lines was cleaved with restriction enzyme EcoT22I, the digested genomic DNA fragments were fractionated by 1% agarose gel electrophoresis, the fractionated genomic DNA fragments were transferred to a nylon membrane, labeled 7H01 was used as a probe for hybridization, and the hybridized bands were detected based on the probe labeling.

Fig. 5 is an image showing EcoT22I/7H01 polymorphism, obtained by genomic analysis of the 11 hop lines.

The results confirmed that 980690 and 981116 are hop lines with only the m-type genome, M916001003, 971174, 980573 and M843501246 are hop lines with only the 1-type genome, and Daimanshu, 0004B, Monica, Hokusenzairai and Maria are hop lines with both the m-type genome and 1-type genome.

The results described above demonstrated that analysis of PshBI/g7H01 polymorphism, like analysis of EcoT22I/7H01 polymorphism, can determine whether or not a hop variety has the m-type genome, thus suggesting that it can be used as a screening method for hop varieties with high α acid contents or hop varieties with high α acid and β acid, myrcene and/or xanthohumol contents.

## Industrial Applicability

According to the invention it is possible to provide a breeding marker for hop varieties with high α acid contents, the breeding marker also being usable as a breeding marker for hop varieties with high α acid contents and high β acid, myrcene and/or xanthohumol contents. According to the invention it is also possible to objectively and rapidly screen for hop varieties with high α acid contents or hop varieties with high α acid, myrcene and/or xanthohumol contents, using hop seeds or seedlings, without waiting for the hops to form cones and develop stabilized traits.

## Claims

1. A breeding marker represented by the following (a) or (b), which is used for screening of hop varieties with high α acid contents.

   (a) A polynucleotide consisting of a nucleotide sequence of 20-1587 continuous nucleotides including nucleotide No.899 of the polynucleotide consisting of the nucleotide sequence as set forth in SEQ ID NO: 5 (wherein the 899th nucleotide is t).
   (b) A polynucleotide consisting of the sequence complementary to the polynucleotide of (a) above.

2. A breeding marker according to claim 1, which is used for screening of hop varieties with high contents of β acids, myrcene and/or xanthohumol.

3. A screening method for hop varieties with high α acid contents, comprising
   an extraction step in which genomic DNA is extracted from a hop variety specimen,
   a digestion step in which the genomic DNA is digested with restriction enzyme EcoT22I to obtain digested genomic DNA fragments,
   a detection step in which the digested genomic DNA fragments are separated and genomic DNA fragments that hybridize to the polynucleotide of (a) or (b) above are detected, and
   a judging step in which it is judged that the variety has a high α acid content if the size of at least one of the genomic DNA fragments is approximately 11.3 kbp.

4. A method for screening of hop varieties with high α acid contents which comprises
   an extraction step in which genomic DNA is extracted from a hop variety specimen, and
   an identifying step in which genomic DNA polymorphism at the 899th nucleotide position of the nucleotide sequence as set forth in SEQ ID NO: 5 is identified.

5. A screening method according to claim 4, wherein tine identifying step comprises
   a PCR step in which DNA is synthesized by PCR using the aforementioned genomic DNA as template, a primer consisting of a nucleotide sequence of 18-50 contiguous nucleotides located toward the 5'-end from the 899th nucleotide of the nucleotide sequence as set forth in SEQ ID NO: 5 and a primer consisting of the sequence complementary to a nucleotide sequence of 18-50 contiguous nucleotides located toward the 3'-end from the 899th nucleotide of the nucleotide sequence as set forth in SEQ ID NO: 5,
   a digestion step in which the DNA is digested with restriction enzyme PshBI to obtain digested DNA fragments, and
   a judging step in which the variety is judged to have a high α acid content if cleavage occurs between the 899th nucleotide and the 900th nucleotide of the nucleotide sequence as set forth in SEQ ID NO: 5.

6. A screening method according to claim 4, wherein the identifying step comprises
   a PCR step in which DNA is synthesized by PCR using the aforementioned genomic DNA as template, a primer including the nucleotide sequence as set forth in SEQ ID NO: 2 and a primer including the nucleotide sequence as set forth in SEQ ID NO: 3,
   a digestion step in which the DNA is digested with restriction enzyme PshBI to obtain digested DNA fragments, and
   a judging step in which the variety is judged to have a high α acid content if the size of at least one of the digested DNA fragments is approximately 460 bp.

7. A screening method according to any one of claims 3 to 6, which is a method for screening of hop varieties with high contents of β acids, myrcene and/or xanthohumol.

8. A primer set to be used for screening of hop varieties with high α acid contents, the primer set comprising
   a primer containing the nucleotide sequence as set forth in SEQ ID NO: 2 and
   a primer containing the nucleotide sequence as set forth in SEQ ID NO: 3.

9. A primer set according to claim 8, which is used for screening of hop varieties with high contents of β acids, myrcene and/or xanthohumol.

*Fig.1*

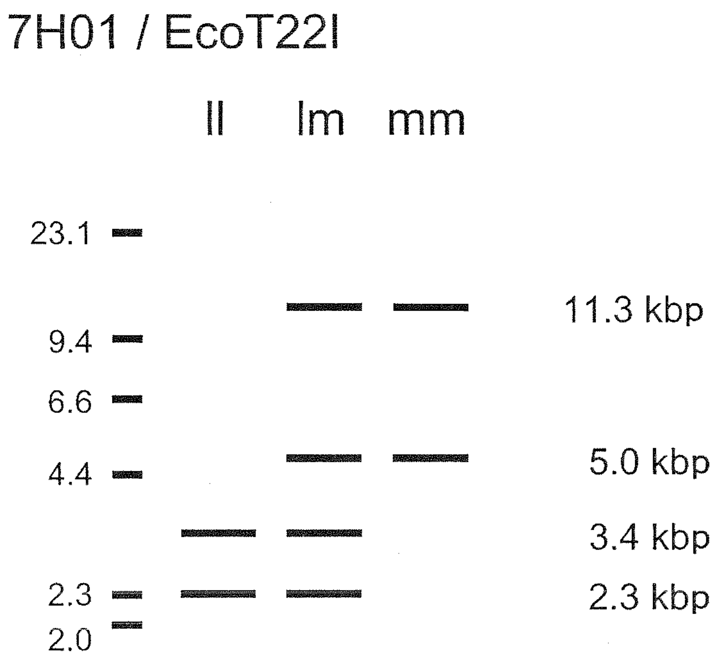
7H01 / EcoT22I
ll
lm
mm
23.1
9.4
6.6
4.4
2.3
2.0
11.3 kbp
5.0 kbp
3.4 kbp
2.3 kbp

## *Fig.2*

```
                                         NO.899
m type  871:TTCACTAATTTGATATCTATTCTAACAATTAATAAATATAATGT 915
l type  864:TTCACTAATTTGATATCTATTCTAACAAATAATAAATATAATGT 908
            **************************** ****************
```

*Fig.3*

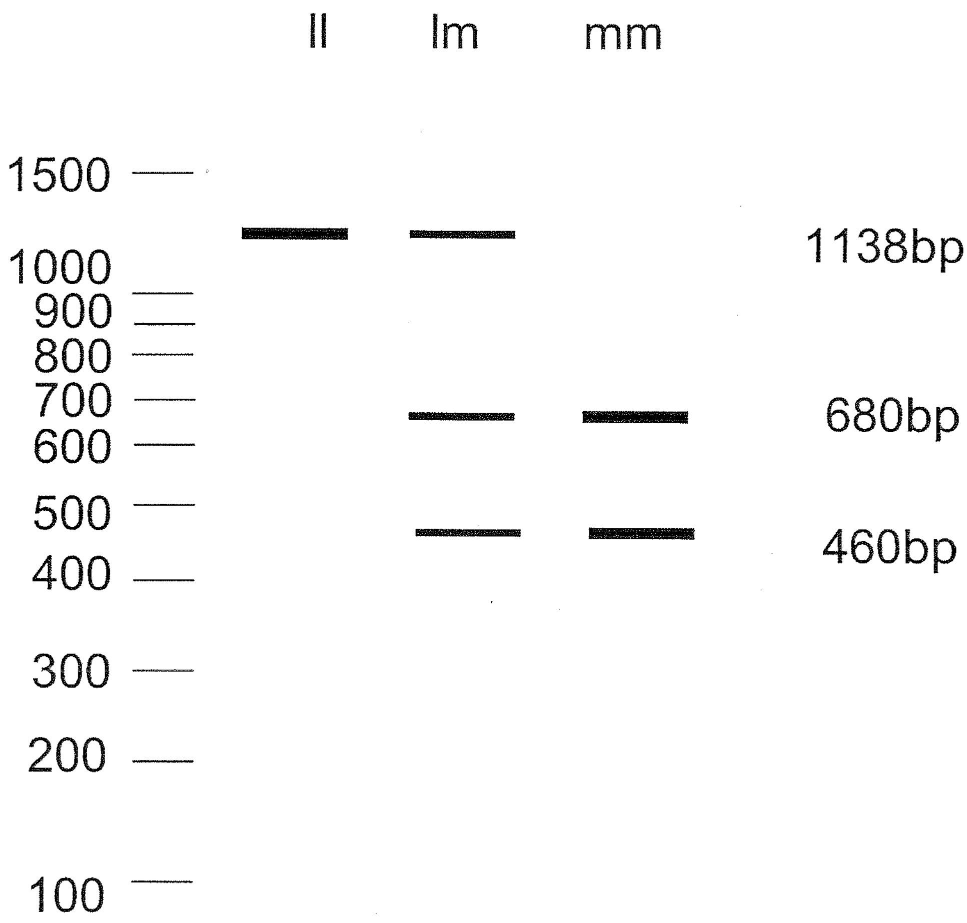
ll
lm
mm
1500
1000
900
800
700
600
500
400
300
200
100
1138bp
680bp
460bp

Fig.4

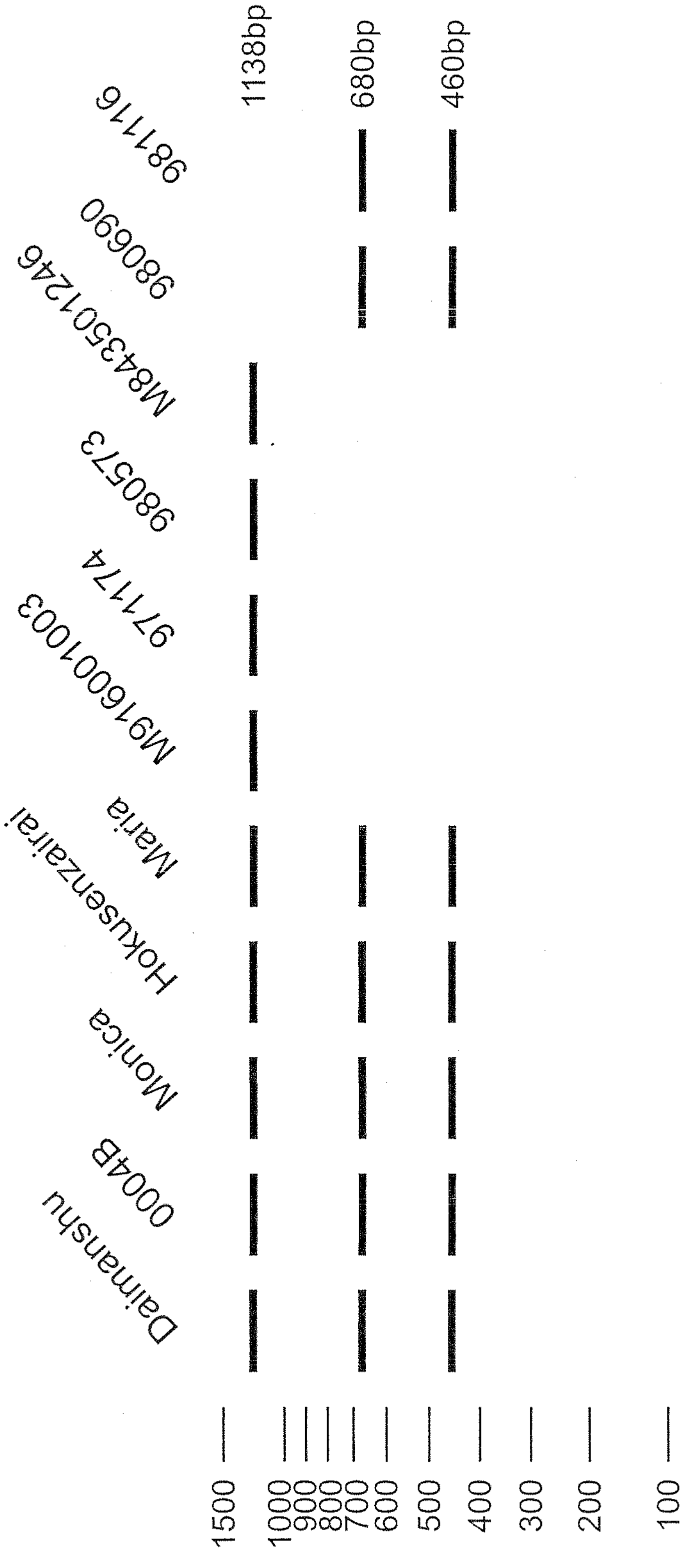

Daimanshu
0004B
Monica
Hokusenzairai
Maria
M91600100
971174
980573
M84350124
980690
981116
1138bp
680bp
460bp
1500
1000
900
800
700
600
500
400
300
200
100

Fig.5

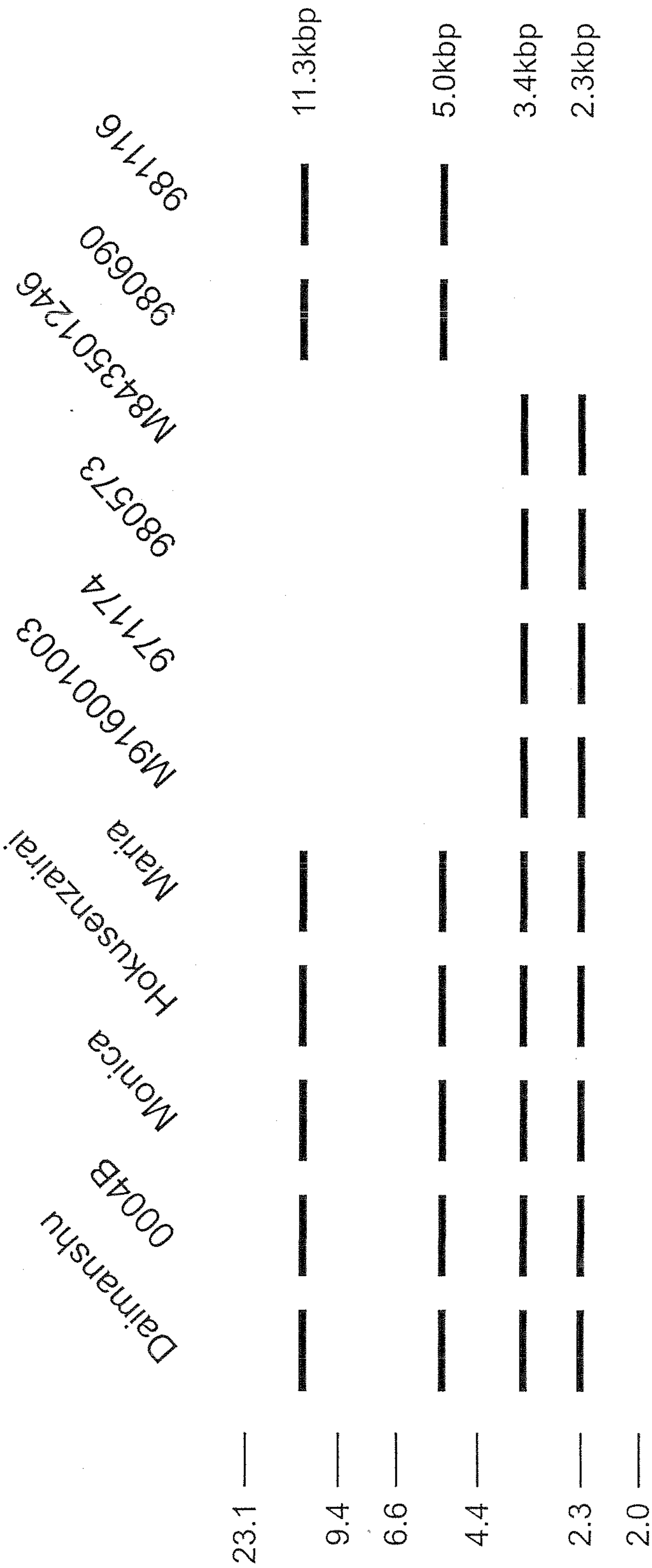
Daimanshu
0004B
Monica
Hokusenzairai
Maria
M916001003
971174
980573
M843501246
980690
981116
11.3kbp
5.0kbp
3.4kbp
2.3kbp
23.1
9.4
6.6
4.4
2.3
2.0

**INTERNATIONAL SEARCH REPORT**

International application No.
PCT/JP2007/068166

A. CLASSIFICATION OF SUBJECT MATTER
*C12N15/09*(2006.01)i, *C12Q1/68*(2006.01)i, *A01H1/04*(2006.01)n

According to International Patent Classification (IPC) or to both national classification and IPC

B. FIELDS SEARCHED

Minimum documentation searched (classification system followed by classification symbols)
C12N15/09, C12Q1/68, A01H1/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
BIOSIS/MEDLINE/WPIDS(STN), CAplus(STN), REGISTRY(STN), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| A | Clarissa Gerhaeuser, et al., European Journal of Cancer, 41(13), 2005, pp.1941-1954, full text | 1-8 |
| A | Hitoshi Aoshima, et al., Journal of Agriculture Food Chemstry, 5(54), 2006, pp.2514-2519, full text | 1-8 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 November, 2007 (01.11.07) | 27 November, 2007 (27.11.07) |
| Name and mailing address of the ISA/ Japanese Patent Office | Authorized officer |
| Facsimile No. | Telephone No. |

Form PCT/ISA/210 (second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Gerhaeuser.** *European Journal of Cancer,* 2005, vol. 41 (13), 1941-1954
- **Aoshima et al.** *J. Agric. Food Chem.,* 2006, vol. 5 (54), 2514-2519
- **Maniatis et al.** Molecular cloning. Cold Spring Harbor Laboratory Press, 1989

SEQUENCE LISTING

<110> Sapporo Breweries Ltd.

<120> Selection method of hop strain, breeding marker for the selection method of hop strain, and primer set

<130> R1546 EP S3

<140> EP 07 82 8261.3
<141> 2007-9-19

<150> JP 2006-254607
<151> 2006-9-21

<160> 5

<170> PatentIn version 3.1

<210> 1
<211> 458
<212> DNA
<213> Humulus lupulus

<400> 1
gtttccggag aatcatggcg gccaattctt ctccgttcaa gataattttg ggatcgtctt 60

cagtagcacg tcggaaaata ttggctgaaa tgggatacga ttttgcaatc atgacagcag 120

acattgacga aaagagtatc cgaaaggaga ttccagaaga gttggttgtg gctcttgctg 180

aggccaaggc agcagccatc cttccaagga tccccactgg tgactacata aatgctgtcg 240

agccaacact gttaattact gcagatcaag tataagcaaa gctctctata tttttgttct 300

cacaatttgt aatgtgttgt atgtgccttt tgaagaaaat tggaaaatag gttattgtcc 360

atgttggttt ttttaccctt agcctttaac aatttagttt cacttgttaa gtagcgtggt 420

aaagtcaatt atgtaggaac tttcttccac tagttttg 458

<210> 2
<211> 20
<212> DNA
<213> Artificial

<220>
<221> Source
<223> /note="Description of artificial sequence: primer"

<400> 2
gtttccggag aatcatggcg 20

<210> 3
<211> 23
<212> DNA
<213> Artificial

<220>
<221> Source
<223> /note="Description of artificial sequence: primer"

```
<400>  3
caaaactagt ggaagaaagt tcc                                               23


<210>  4
<211>  1580
<212>  DNA
<213>  Humulus lupulus

<400>  4
gtttccggag aatcatggcg gccaattctt ctccgttcaa ggtagtgctt gtaattagga      60

cttattcctt ctctcctatg taaatttccg aaaggaacga tttacatgct tctttgcgag     120

atcgagagaa tattaagtgt tctgtttgat tggtagtttc tgttctacca gtaaattgtc     180

gaagtaactg gcgtttattg tgtgcagata attttgggat cgtcttcagt agcacgtcgg     240

aaaatattgg ctgaaatggg atacgatttt gcaatcatgg taaaatttct gattgtcttt     300

ttttggtttt gaattttctg ggaacttcag tgtttctact tgtagactgc agacattgac     360

gaaaagagta tccgaaagga gattccagaa gagttggttg tggctcttgc tgaggccaag     420

gtcaggtttt aaatttgata gtgtctagtt gatttgataa gttagtgccg gttggtaagg     480

tcttgttgtt attctttctt tttttccccc ttttttattg ggtgtctgct ttctctgctt     540

tgggcttgag ttcaagtatt ttccgttgat tacatatcaa ttactcactc aggctgaagc     600

catcatatca aaattagaat ctttggataa tcaaaacaag gatgacaacg caacattatt     660

gattgcagcg gatacagtat gtctttttgt tttcttcatt ttaggcatat ttttaaaaaa     720

aaaaagatgg tgtcacctag ggccatttat ggatggagaa tgcatttcat ttttaacaag     780

gatacgtttt tgctcaatca atatctgggg cattgtctta tgagtaatgc taaagacaaa     840

catgattata cccaacgttt acattcacta atttgatatc tattctaaca aataataaat     900

ataatgtgat ttgaaaaaaa acataagtga tcgagtagca ggtcccattt attgtttgtg     960

atttttttgt ctggattctt ccatttattt ctgtcttaaa aaaatatcct caattgacaa    1020

aatatttaag atcatttgtt cctagtttgg tatttcagga ggggtttttt ttttttttggg    1080

ccgaaactaa gctgcttcaa gtagcgtttg tgctccttgc taccagttta ctccatgttt    1140

agttggtagt aaatatactg ggtatttatt agggtcctag ctctacagtt agacactacg    1200

tctttgtagt ttatactaat ggtaatcata ttattgttct tttccaagca ttactagtct    1260

ggttgtagtg taactgtgtt ggcacttacc tggtggaaac ttcttctcag gcagcagcca    1320

tccttccaag gatccccact ggtgactaca taaatgctgt cgagccaaca ctgttaatta    1380

ctgcagatca agtataagca aagctctcta tatttttgtt ctcacaattt gtaatgtgtt    1440

gtatgtgcct tttgaagaaa attggaaaat aggttattgt ccatgttggt ttttttaccc    1500

ttagccttta acaatttcgt ttcacttgtt aagtagcgtg gtaaagtcaa ttatgtagga    1560

actttcttcc actagttttg                                                1580
```

<210> 5
<211> 1587
<212> DNA
<213> Humulus lupulus

<400> 5
gtttccggag aatcatggcg gccaattctt ctccgttcaa ggtagtgctt gtaattagga 60

cttattcctt ctctcctatg taaatttctg aaaggaacga tttacatgct tctttgcgag 120

atcgagagaa tattaagtgt tctgtttgat tgatagttta gtttctgttc taccagtaaa 180

ttgtcgaagt aactggcgtt tattgtgtgc agataatttt gggatcgtct tcagtagcac 240

gtcggaaaat attggctgaa atgggatacg attttgcaat catggtaaaa tttctgattt 300

ttttttttt ggttttgaat tttctgggaa cttcagtgtt tctacttgta gacagcagac 360

attgacgaaa agagtatccg aaaggagatt ccagaagagt tggttgtggc tcttgctgag 420

gccaaggtca ggttttaaat ttgatagtgt ctagttgatt tgataagtta gtgccggttg 480

gtaaggtctt gttgttattc tttctttttt tccccctttt ttattgggtg tctgctttct 540

ctgctttggg cttgagttca aatattttcc gttgattaca tatcaattac tcactcaggc 600

tgaagccatc atatcaaaat tagaatcttt ggataatcaa aacaaggatg acaacccaac 660

attattgatt gcagcggata cagtatgtct ttttgttttc ttcattttag gcatattttc 720

aaaaaaaaaa aagatggtgt cacctagggc catttatgga tggagaatgc atttcatttt 780

taacaaggat acgtttttgc tcaatcaata gctggggcat tgtcttatga gtaatgctaa 840

agacaaacat gattataccc aacatttaca ttcactaatt tgatatctat tctaacaatt 900

aataaatata atgtggtttg aaaaaaaaca taagtgatcg agaagcaggt cccatttatt 960

gtttgtgatt cttttgtctg gattcttcca tttatttctg tcttaaaaaa atatcctcaa 1020

ttgacaaaat atttacgatc atttgttcct agtttggtat ttcaggaggg tttttttttt 1080

tttggggccg aaactaagct gcttcaagta gcgtttgtgc tccttgctac cagtttactc 1140

catgtttagt tggtagtaaa tatactgggt atttattagg gtcctagctc tacagttaga 1200

cactacgtct ttgtagttta tactaatggt aatcatatta ttgttctttt ccaagcatta 1260

ctagtctggt tgtagtgtaa ctgtgttggc acttacctgg tggaaacttc ttctcaggca 1320

gcagccatcc ttccaaggat ccccactggt gactacataa atgctgtcga gccaacactg 1380

ttaattactg cagatcaagt ataagcaaag ctctctatat ttttgttctc acaatttgta 1440

atgtgttgta tgtgcctttt gaagaaaatt ggaaaatagg ttattgtcca tgttggtttt 1500

tttaccctta gcctttaaca atttagtttc acttgttaag tagcgtggta aagtcaatta 1560

tgtaggaact ttcttccact agttttg 1587